(19) Europäisches Patentamt / European Patent Office / Office européen des brevets

(11) EP 4 407 624 B1

(12) EUROPEAN PATENT SPECIFICATION

(45) Date of publication and mention
of the grant of the patent:
**31.12.2025 Bulletin 2026/01**

(21) Application number: **23207229.8**

(22) Date of filing: **01.11.2023**

(51) International Patent Classification (IPC):
**G16B 25/10** (2019.01)    **G16B 40/20** (2019.01)

(52) Cooperative Patent Classification (CPC):
**G16B 25/10; G16B 40/20;** G16B 5/00

(54) **METHOD AND SYSTEM FOR DESIGNING DRUG-LIKE MOLECULES FROM DESIRED GENE EXPRESSION SIGNATURES**

VERFAHREN UND SYSTEM ZUM ENTWURF VON ARZNEIMITTELÄHNLICHEN MOLEKÜLEN AUS GEWÜNSCHTEN GENEXPRESSIONSSIGNATUREN

PROCEDE ET SYSTEME DE CONCEPTION DE MOLECULES DE TYPE MEDICAMENT A PARTIR DE SIGNATURES D'EXPRESSION GENIQUE SOUHAITEES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **30.01.2023 IN 202321005947**

(43) Date of publication of application:
**31.07.2024 Bulletin 2024/31**

(73) Proprietor: **Tata Consultancy Services Limited Maharashtra (IN)**

(72) Inventors:
• **DAS, DIBYAJYOTI**
  **500081 Hyderabad, Telangana (IN)**
• **ROY, ARIJIT**
  **500081 Hyderabad, Telangana (IN)**
• **SRINIVASAN, RAJGOPAL**
  **500081 Hyderabad, Telangana (IN)**
• **CHAKRABARTY, BROTO**
  **500081 Hyderabad, Telangana (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**US-A1- 2020 365 238**

• **BORN JANNIS ET AL: "PaccMann^RL: De novo generation of hit-like anticancer molecules from transcriptomic data via reinforcement learning", vol. 24, no. 7, 5 March 2021 (2021-03-05), US, XP093148954, ISSN: 2589-0042, Retrieved from the Internet <URL:https://doi.org/10.1016/j.isci.2021.102269> [retrieved on 20240408], DOI: 10.1016/j.isci**
• **HONGYUAN DONG ET AL: "Variational Autoencoder for Anti-Cancer Drug Response Prediction", ARXIV.ORG, CORNELL UNIVERSITY LIBRARY, 201 OLIN LIBRARY CORNELL UNIVERSITY ITHACA, NY 14853, 18 September 2020 (2020-09-18), XP081765702**

**Description**

TECHNICAL FIELD

The disclosure herein generally relates to the field of drug discovery and development and more particularly, to a method and system for designing drug-like molecules from desired gene expression signatures.

BACKGROUND

Drug induced gene expression profiling provides useful information covering various aspects of drug discovery and development. Most importantly, this knowledge can be used to discover drugs mechanism of actions. Recently, deep learning-based drug design methods are in spotlight due to their ability to explore huge chemical space and design property optimized target-specific drug molecules. Recent advances in accessibility of open-source drug-induced transcriptomic data along with the ability of deep learning algorithms to understand hidden patterns have opened opportunities for designing drug molecules based on desired gene expression signatures.

In recent times, number of deep learning based generative models have emerged, which showed promise to accelerate the whole drug discovery process. The deep learning-based generative methods can explore novel molecules from available chemical space, design target-specific de novo molecules, perform on-the-fly property optimization. However, there are lack of methods that can design molecules based on disease phenotype alone. In this context, document "PaccMann^RL: De novo generation of hit-like anticancer molecules from transcriptomic data via reinforcement learning", JANNIS ET AL, DOI: 10.1016/j.isci, discloses a method of training a variational autoencoder (VAE) to learn the syntax of molecular SMILES representations and a VAE for embedding gene expression profiles from cancer patients, before jointly retraining them. During joint retraining, reinforcement learning is used with the aim to generate molecules with efficacy against a target gene expression profile. The document does not disclose using cell-specific gene expression profiles, nor the use of a joint loss function composed of cross entropy loss and Kullback-Leibler divergence loss which is backpropagated to a decoder that learns conditional mapping of a molecular space to a transcriptomics space in a cell-specific manner.

SUMMARY OF THE INVENTION

The invention is set out in the appended claims.

SUMMARY OF RELATED DISCLOSURE

Embodiments of the disclosure present technological improvements as solutions to one or more of the above-mentioned technical problems recognized by the inventors in conventional systems. For example, in one embodiment, a method and system for designing drug-like molecules from desired gene expression signatures is provided.

In one aspect, a processor-implemented method for designing drug-like molecules from desired gene expression signatures is provided. The processor-implemented method comprising receiving, via an input/output interface, a gene expression profile in a cell-specific manner as an input and a dataset of molecules from a predefined drug-like small molecule database. Further, the processor-implemented method includes pre-processing, via one or more hardware processors, the received dataset of molecules to obtain a training dataset of molecules, wherein the molecules, represented by Simplified Molecular Input Line Entry System (SMILES) and within a predefined length are considered. Furthermore, the processor-implemented method includes training, via one or more hardware processors, a simplified molecular input line entry system (SMILES) variational autoencoder (s-VAE) and a profile variational autoencoder (p-VAE) jointly with the obtained training dataset of molecules. Finally, the method includes generating, via the one or more hardware processors, one or more conditional novel small molecules in SMILES format from the received gene expression profile using jointly trained s-VAE and p-VAE.

In another aspect, a system for designing drug-like molecules from desired gene expression signatures is provided. The system includes at least one memory storing programmed instructions, one or more Input /Output (I/O) interfaces, and one or more hardware processors operatively coupled to the at least one memory, wherein the one or more hardware processors are configured by the programmed instructions to receive a gene expression profile in a cell-specific manner as an input and a dataset of molecules from a predefined drug-like small molecule database. Further, the one or more hardware processors are configured by the programmed instructions to pre-process the received dataset of molecules to obtain a training dataset of molecules, wherein the molecules represented by Simplified Molecular Input Line Entry System (SMILES) and within a predefined length, are considered. Furthermore, the one or more hardware processors are configured by the programmed instructions to train a simplified molecular input line entry system (SMILES) variational autoencoder (s-VAE) and a profile variational autoencoder (p-VAE) jointly with the obtained training dataset of molecules. Finally, the one or more hardware processors are configured by the programmed instructions to generate conditional novel small molecules in SMILES format from the received gene expression profile using jointly trained s-VAE and p-VAE.

In yet another aspect, one or more non-transitory machine-readable information storage mediums are provided comprising one or more instructions, which when executed by one or more hardware processors causes a method for designing drug-like molecules from desired gene expression signatures. The processor-implemented method comprising receiving, via an input/output interface, a gene expression profile in a cell-specific manner as an input and a dataset of molecules from a ChEMBL database. Further, the processor-implemented method includes pre-processing, via one or more hardware processors, the received dataset of molecules to obtain a training dataset of molecules, wherein the molecules represented by Simplified Molecular Input Line Entry System (SMILES) and within a predefined length, are considered. Furthermore, the processor-implemented method includes training, via one or more hardware processors, a simplified molecular input line entry system (SMILES) variational autoencoder (s-VAE) and a profile variational autoencoder (p-VAE) jointly with the obtained training dataset of molecules. Finally, the method includes generating, via the one or more hardware processors, conditional novel small molecules in SMILES format from the received gene expression profile using jointly trained s-VAE and p-VAE.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a network diagram of a system for designing drug-like molecules from desired gene expression signatures, in accordance with some embodiments of the present disclosure.
FIG. 2 is an exemplary flow diagram illustrating a method for designing drug-like molecules from desired gene expression signatures, in accordance with some embodiments of the present disclosure.
FIG. 3 illustrates a block diagram to illustrate a validation of the pre-trained deep learning model against a disease profile, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Drug discovery is a time-consuming, cost-intensive, and high-risk venture lasting over years from target identification to market approval. Drug discovery usually starts with a well-defined hypothesis about the disease, followed by target identification. Once a suitable target is identified, usually ligand-based or structure-based drug design methods are used for hitting identification. **In** the next subsequent stages, the identified hits go through various pre-clinical and clinical trial stages where drug toxicity, safety and efficacy are further tested. **In** most cases, little is known about the drug mechanism of action. The cumulative effect of all these factors contribute to high failure rates of 90% for drugs in the clinical trial stages alone. The failure rate is even higher in the pre-clinical stages.

Gene-expression has become a useful tool for drug discovery and development as it can capture the molecular signature of a disease and its relationship with phenotypic environment. Gene expression can also identify probable drug targets. The drug induced gene expression is useful to understand how drug molecules target selected cellular pathways or avoid cellular pathways that lead to toxic effects. **In** recent times, it is a standard practice to perform drug induced gene expression analysis on the target cell lines to understand the effect of drugs on cellular pathways, their dose dependence and check pharmacodynamic properties. Several groups have used gene expressions to understand drug-dose response, drug safety, response of specific pathways and identify off-target side-effects.

Inclusion of gene-expression during initial stage of the drug-design can potentially resolve the problem of late-stage attrition of drug molecules and thereby it can greatly reduce the time and cost of the overall drug discovery and development. Moreover, gene expression based omic approach can generate novel molecules without the need for prior knowledge about target-specific ligand dataset that are used for ligand-based drug discovery or three-dimensional structures of the target proteins that are used for structure-based drug design.

In recent times, number of deep learning based generative models have emerged, which showed promise to accelerate the whole drug discovery process. The deep learning-based generative methods can explore novel molecules from available chemical space, design target-specific de novo molecules, perform on-the-fly property optimization. However, there are lack of methods that can design molecules based on disease phenotype alone. Gene expression provides useful information like drug mechanism of action. Such a method does not require target information and provide important information like off-target effects, which is a major reason for late stage drug attrition.

Therefore, embodiments herein provide a method and system for designing drug-like molecules from desired gene expression signatures. Initially, a gene expression profile is received in a cell-specific manner as an input, via an input/output interface, and a dataset of molecules from a predefined drug-like small molecule database such as ChEMBL. A pre-trained deep learning model comprises of a pretrained profile variational autoencoder (pVAE), which learns to project gene expression profiles into a latent space, which is then passed through a pre-trained SMILES variation autoencoder. After the completion of the combined training of pVAE and sVAE, a desired gene expression is supplied to the pre-trained deep learning model for conditional generation of novel small molecule in SMILES format. The pre-trained deep learning model is tested against three breast cancer target genes (using gene knocked out expression profiles of the respective genes), where it could design novel inhibitors with high bioactivity. Finally, the pre-trained deep learning model is tested against triple negative breast cancer profile (TNBC), a high-risk aggressive cancer, where the pre-trained deep learning model could design novel molecules similar to known inhibitors. It is to be noted that an in-silico validation is performed for both the cases, where designed molecules are found to be highly similar to existing inhibitors.

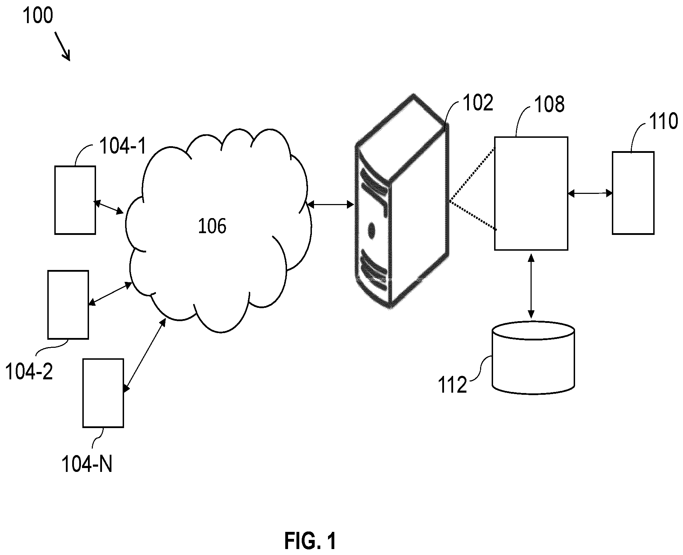

Referring now to the drawings, and more particularly to FIG. 1 through FIG. 3, where similar reference characters denote corresponding features consistently throughout the figures, there are shown preferred embodiments and these embodiments are described in the context of the following exemplary system and/or method.

FIG. 1 illustrates a network diagram of a system 100 for designing drug-like molecules from desired gene expression signatures. Although the present disclosure is explained considering that the system 100 is implemented on a server, it may also be present elsewhere such as a local machine. It may be understood that the system 100 comprises one or more computing devices 102, such as a laptop computer, a desktop computer, a notebook, a workstation, a cloud-based computing environment and the like. It will be understood that the system 100 may be accessed through one or more input/output interfaces 104-1, 104-2... 104-N, collectively referred to as I/O interface 104. Examples of the I/O interface 104 may include, but are not limited to, a user interface, a portable computer, a personal digital assistant, a handheld device, a smartphone, a tablet computer, a workstation and the like. The I/O interface 104 are communicatively coupled to the system 100 through a network 106.

In an embodiment, the network 106 may be a wireless or a wired network, or a combination thereof. In an example, the network 106 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 106 may include a variety of network devices, including routers, bridges, servers, computing devices, storage devices. The network devices within the network 106 may interact with the system 100 through communication links.

The system 100 may be implemented in a workstation, a mainframe computer, a server, and a network server. In an embodiment, the computing device 102 further comprises one or more hardware processors 108, one or more memory 110, hereinafter referred as a memory 110 and a data repository 112, for example, a repository 112. The data repository 112 may also be referred as a dynamic knowledge base 112 or a knowledge base 112. The memory 110 is in communication with the one or more hardware processors 108, wherein the one or more hardware processors 108 are configured to execute programmed instructions stored in the memory 110, to perform various functions as explained in the later part of the disclosure. The repository 112 may store data processed, received, and generated by the system 100. The memory 110 further comprises a plurality of modules. The plurality of modules is configured to perform various functions.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

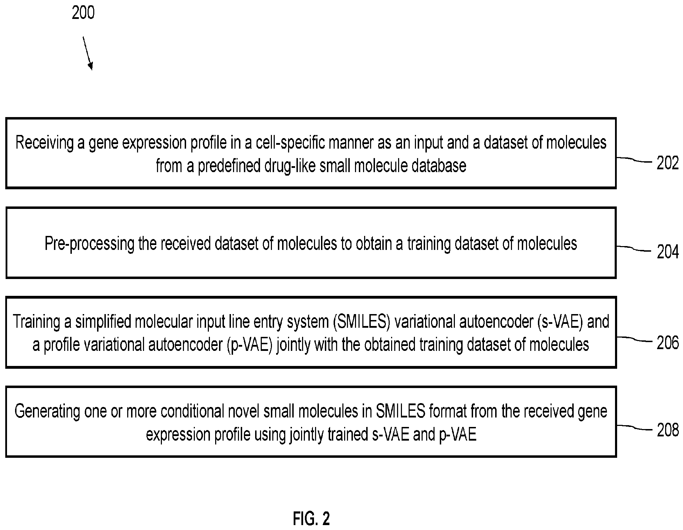

FIG. 2 is an exemplary flow diagrams illustrating a processor-implemented method 200 for designing drug-like molecules from desired gene expression signatures implemented by the system of FIG. 1, according to some embodiments of the present disclosure.

Initially at step 202 of the method 200, a gene expression profile in a cell-specific manner is received, via an input/output interface 104, as an input and a dataset of molecules from a predefined drug-like small molecule database such as a ChEMBL. The gene expression profile includes molecular signature of a disease and its relationship with a phenotypic environment.

At the next step 204 of the method 200, the one or more hardware processors 102 are configured by the programmed instructions to preprocess the received dataset of molecules to obtain a training dataset of molecules, wherein the molecules with a predefined length are considered.

At the next step 206 of the method 200, the one or more hardware processors 102 are configured by the programmed instructions to train a simplified molecular input line entry system (SMILES) variational autoencoder (s-VAE) and a profile variational autoencoder (p-VAE) jointly with the obtained training dataset of molecules. The simplified molecular input line entry system (SMILES) is a string-based representation of the predefined molecules. It would be appreciated that the generating SMILES from gene expression data is considered as a mapping function of the molecular structure to gene expressions. The predefined molecules are represented in the SMILES format, where the system 100 learns grammar of the predefined molecules using the s-VAE.

In one illustration, the dataset of molecules for training the s-VAE model is obtained from the ChEMBL database. This dataset is pre-processed to obtain the final training dataset with ~1.6 million molecules. The molecules with length smaller than 100 is only considered. Filters are applied to remove stereochemistry, salts, and molecules with undesirable atoms or groups and the remaining molecules are canonicalized as described in our earlier works. The molecules from both ChEMBL and L1000 CDS2 are represented in the SMILES format to leverage the effectiveness of deep learning networks. SMILES is a string-based representation of the molecules based on the principles of molecular graph theory.

This allows molecular structure depiction with straightforward rules and has been used in several papers representing molecular information in-silico. During the pre-training, only molecules from ChEMBL dataset is used so that the model can learn the grammar of small molecules from a bigger dataset and design diversified novel small molecules. The deep neural network architecture of the s-VAE model consists of an encoder and a decoder. Both encoder and decoder consist of two layers of 1024 bidirectional gated recurrent units (GRU) as the internal memory, augmented with a stack acting as the dynamic external memory. The stack had a width of 256 units and a depth of 105 units. An embedding layer and a dense layer with log softmax activation are used to pass the input to the encoder and retrieve the output from the decoder, respectively.

In one example, wherein the L1000 dataset is used to obtain the mRNA gene-expression profiles which are generated as part of the Library of Integrated Network-based Cellular Signatures (LINCS). The L1000 contains gene perturbations including chemical, gene knockouts and overexpression. The L1000 gene expression profiles calculated using the characteristic direction (CD) method are used for all the analyses. The CD data set has been shown to be highly sensitive to differential gene expression. To further refine the input data, a high-quality CD dataset is extracted. The high-quality data is filtered based on the p-value $\leq 0.05$ across replicates. The p-value is a measure of reliability of the data obtained based on the expression values of the replicates of the experiment. Lower p-values indicate that the expression values are consistent across the replicates and that the probability of obtaining the values by random chance is low. MCF7, PC3 and VCAP cell lines had maximum high-quality data available. The perturbagen dosage concentration of 10 $\mu$M is administered most across the experiments and the time points of 6h and 24h are most frequently observed. Based on the availability of the high-quality data, the combination of MCF7 cell line, 10 $\mu$M concentration and 24h time points are considered for building the model as this consisted of the maximum number of available datasets. For each expression profile, the induced drug is represented in SMILES format, pre-processed, filtered and canonicalized, resulting in 1,404 data points. This is randomly split at a 9:1 ratio for train and test set. The training data is used to update the weights of the VAE whereas the test data was never shown to the model during training.

In another embodiment, an encoder of the p-VAE learns to project the received gene expression profiles into a latent space. The pVAE consisted of an encoder with an input layer of 978, two hidden layers of size 768 and 512, and two vectors of 256 dimensions describing the mean and variance of the latent space distributions. The dimensionality of the latent space was kept at 256 for seamless transfer to the SMILES Variational. Autoencoder which was pretrained with a latent space of 256 dimensions. The dimensions of the interim layers were carefully chosen after several trials, where the aim was to select combinations with minimum loss. This latent space is the compressed knowledge representation of the gene expression data and is therefore the most important part of the network. The encoder generates a latent vector by sampling from defined mean and variance of the distributions and proceeds to develop a reconstruction of the original gene expression input. The decoder is the inverse of the encoder with input layers from 256 to an output of 978 via 512 and 768 nodes.

The reconstruction of the gene expression is considered as a regression problem and the loss of the auto encoder is measured by Mean squared error and Kullback-Leibler Divergence (KLD) using the formulae:

$$Loss = \alpha \times E + (1 - \alpha) \times \beta \times KLD \qquad (1)$$

wherein $\alpha$ and $\beta$ coefficients are considered as 0.5 and 1.0 respectively and E as mean squared error where KLD or Kullback-Leibler Divergence indicates the dissimilarity between two distributions and is defined as

$$KLD(P||Q) = \int_{-\infty}^{\infty} p(x)\log\left(\frac{p(x)}{q(x)}\right) dx \qquad (2)$$

where P represents the Gaussian distribution of the compound and Q represents the standard polynomial Gaussian distribution. The probability density function of P and Q were given by p(x) and q(x). The model was trained by minimizing equation 1, for 500 epochs using Adam optimizer and an initial learning rate of 1e-5. The dataset was split into minibatch sizes of 32 each.

Referring back to FIG. 2, at the last step 208 of the method 200, the one or more hardware processors 102 are configured by the programmed instructions for conditional novel small molecules generation in SMILES format from the received gene expression profile using jointly trained s-VAE and p-VAE. The designed conditional novel small molecule is pass through one or more physico-chemical filters to satisfy one or more drug-like properties. The generated conditional novel small molecule induces a desired gene expression.

The combined model consists of the p-VAE encoder and the s-VAE decoder. The pretraining of the p-VAE ensured that it is capable of extracting essential features of the input gene expression while the pretrained s-VAE ensured better learning of the SMILES grammar which can generate more diverse molecules. The combined model is trained with CD values of L1000 gene expression data where the output is small molecules in SMILES representation. The model is trained for 500 epochs with the backpropagation limited only to the SMILES decoder. The small molecules and their corresponding induced gene expression data are used for the joint training of the model. As mentioned earlier, maximum amount of high-quality data could be extracted from the MCF7 cell line. The combinations of MCF7 cell line, 10 μM concentration and 24h time point is considered for further model building. The data is divided into 90% training data and 10% test data. This data is fed to the model in batches of 64 with a Kullback-Leibler growth rate of 0.05 and a learning rate of 1e-3. The model is trained to minimize a joint loss function, as in equation (1), with E as cross entropy loss (CE) of the adjacency matrix reconstruction and the Kullback-Leibler divergence (KLD) loss to enforce the latent variables to follow the Gaussian distribution.

It would be appreciated that the validation of the pre-trained deep learning model is carried out in two ways:

(i) The desired gene expression due to gene knockdown is collected, where each of the knocked down gene is assumed to be disease associated gene. The known inhibitors of these genes are collected and compared with the designed molecules.

(ii) The disease gene expression profile is directly used to design molecules against the disease of interest. The CRISPR gene knockdown data is extracted from the SigCom LINCS L1000 CDS2 portal.

The available CRISPR knocked out profiles are extracted, and average Pearson Correlation Coefficient (PCC) is calculated for each knocked out gene. Most genes have multiple profiles and only high-quality profiles are considered for the current study. The profiles where the average PCC is greater than 0.7 is considered as high confidence profiles for further analyses. The single profile available for AURKA, a well-known anti-breast cancer gene target, is also considered.

Further, the molecules known to inhibit the respective human genes whose knocked out profiles are extracted, are searched from a ExCape database. The molecules with pXC50 values of at least 5 or greater are considered as inhibitors for the given genes. The active molecules are converted to SMILES format, canonicalized and only with length less than 100 are stored as the validation dataset. Only those genes with more than 100 inhibitors are considered. If any of the small molecule from the validation dataset is present in the training dataset, they are removed from the list. This resulted in 2,316, 1,057 and 111 known inhibitors for AURKA, ADRB3 and PSMB5 respectively. The list of known inhibitors from the validation dataset, and the newly designed molecules are compared for in silico validation.

Further, to validate the effectiveness of the pre-trained deep learning model, generated molecules are compared with known inhibitors. Several generated molecules are found to be highly similar compared to the known inhibitors. The generated molecules also displayed a high internal diversity. The generated molecules are tested for their physio-chemical properties specifically for drug likeness, partition coefficient, molecular weight and found to be drug-like. The pre-trained deep learning model is further tested against a gene expression profile and is able to recall known breast cancer drugs. Overall, the pre-trained deep learning model is able to sample from the diverse chemical space and generated several potent drug-like compounds.

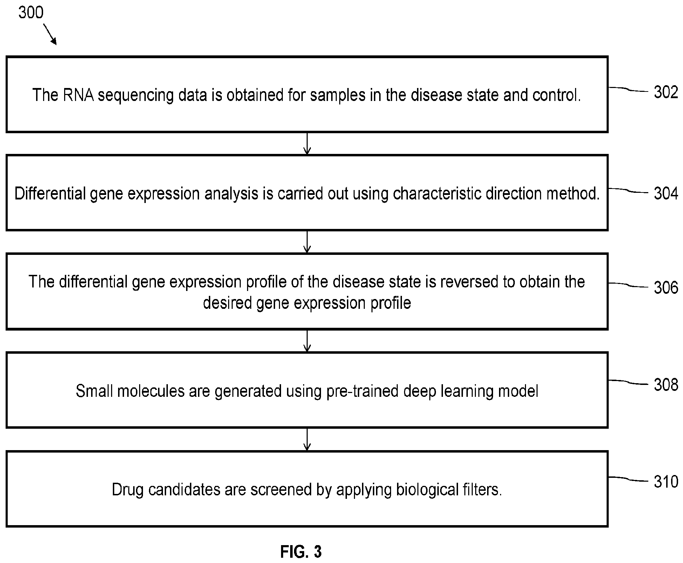

FIG. 3 is a flow diagram illustrating a validation of the pre-trained deep learning model against a disease profile, according to some embodiments of the present disclosure. Initially, Ribonucleic acid (RNA) sequencing data is obtained for samples in the disease state and control (302). Further, a differential gene expression analysis is carried out using characteristic direction method (304). The differential gene expression profile of the disease state is reversed to obtain the desired gene expression profile (306). Small molecules are generated using the pre-trained deep learning model also referred as Gex2SGen (308). Finally, drug candidates are screened by applying one or more predefined biological filters (310).

In one example, wherein a validation of the pre-trained deep learning model is performed against a disease profile from a triple negative breast cancer (TNBC). The gene expression signature for TNBC is collected from a high throughput sequencing (GSE113230), where ribosomal RNA-depleted total RNA was extracted and sequenced from three pairs of triple-negative breast cancer samples and adjacent normal tissues. The corresponding characteristic direction (CD) profile was obtained from SigCom LINCS portal. The desired effect of an anti-breast cancer drug is expected to reverse the differential expression of the genes in the disease state. Therefore, the inverse TNBC profile is obtained by transforming the expression values in the reverse direction. The values of the expressions are inversed by multiplying with negative one. The inverse TNBC cancer profile is considered as the desired gene expression input for the pre-trained deep learning model. The existing drugs against breast cancers are extracted from the DrugBank and compared against conditionally generated molecules by the model based on the desired profile.

Various physiochemical properties of the molecules are calculated using rdkit package implemented in python. These physicochemical properties include Quantitative Estimate of Drug-likeness (QED), partition-coefficient (LogP), molecular weight (MW), number of hydrogen bond donors, number of hydrogen bond acceptor, molecular polar surface area, number of rotatable bonds, number of aromatic rings and the number of structure alerts. QED is based on the underlying distribution on eight molecular properties i.e., Molecular weight, octanol water partition coefficient, number of hydrogen bond donors, number of hydrogen bond acceptor, molecular polar surface area, number of rotatable bonds, number of aromatic rings and the number of structure alerts. As the QED values move towards 1, the more compounds can be considered as druglike. LogP not greater than 5, molecular weight not greater than 500, no more than 5 hydrogen bond acceptors and no more than 10 hydrogen bond donors were considered as other property filters to satisfy Lipinski's Rule of 5.

Physicochemical properties of the generated molecules: The 10,000 molecules generated for each the gene-knocked out profile are further examined for certain drug like properties (as described in the method section). The drug-likeness distribution of the generated molecules is found to be significantly better than the known inhibitors. The peak of the QED distribution is around 0.5 for the known inhibitors of AURKA, whereas for the generated molecules, the peak is around 0.8. In case of ADRB3 and PSMB5, the peak of the QED distribution for generated molecules is around 0.8, whereas the peak for the known inhibitors is around 0.2. Similarly, the logP, molecular weight, hydrogen bond acceptor and hydrogen bond donor distribution of the generated molecules are significantly better for the generated molecules, compared to known inhibitors. After applying the property filters, 3940, 3465 and 3463 molecules were obtained from gene knocked out profiles of AURKA, ADRB3 and PSMB5 respectively.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address the problem of generating molecules from the knocked-out gene expression profiles which are significantly unrelated (having low Tanimoto coefficients) with the known inhibitors of the genes. Embodiments herein provide a method and a system for a cell specific model where gene expressions are processed via a pretrained SMILES variational autoencoder to produce new molecules. The model is trained with the drug induced gene expression data as the input. Both the pretrained SMILES VAE and pVAE are trained jointly. During the joint training, the difference between the newly generated molecules and the existing drug molecules is calculated as a joint loss function composed of binary cross entropy loss and the Kullback-Leibler divergence (KLD) loss. This loss is backpropagated to the decoder part of the model, which helped the model accurately learn the conditional mapping of the molecular space to the transcriptomic space in a cell-specific manner. The method disclosed design drug-like small molecules from the desired gene expression profile, using two variational auto encoders, which jointly learnt the small molecules and their corresponding gene expression profiles.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs, GPUs etc.

The embodiments herein can comprise hardware and software elements. The embodiments that are implemented in software include but are not limited to, firmware, resident software, microcode, etc. The functions performed by various components described herein may be implemented in other components or combinations of other components.

For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200) comprising:

   receiving (202), via an input/output interface (104), a gene expression profile in a cell-specific manner as an input and a dataset of molecules from a predefined drug-like small molecule database;
   pre-processing (204), via one or more hardware processors (108), the received dataset of molecules to obtain a training dataset of molecules with a predefined length, wherein the received dataset of molecules is pre-processed using filters to remove stereochemistry, salts, and molecules with undesirable atoms;
   jointly training (206), via the one or more hardware processors (108), a simplified molecular input line entry system (SMILES) variational autoencoder (s-VAE) and a profile variational autoencoder (p-VAE) with the obtained training dataset of molecules, wherein the training dataset of molecules are represented in a SMILES format where grammar of the training dataset of molecules is learnt using the s-VAE; and
   generating (208), via the one or more hardware processors (108), one or more conditional novel small molecules in SMILES format from the received gene expression profile using trained s-VAE and p-VAE, wherein during the joint training, a difference between the one or more conditional novel small molecules and the obtained training dataset of molecules is calculated as a joint loss function composed of binary cross entropy loss and Kullback-Leibler divergence (KLD) loss and wherein the joint loss function is backpropagated to a decoder that learns conditional mapping of a molecular space to a transcriptomic space in a cell-specific manner.

2. The processor-implemented method (200) of claim 1, wherein the gene expression profile reflects molecular signature of a disease.

3. The processor-implemented method (200) of claim 1, wherein the simplified molecular input line entry system (SMILES) is a string-based representation of the molecules.

4. The processor-implemented method (200) of claim 1, wherein an encoder of the p-VAE learns to project the received gene expression profiles into a latent space, and wherein the decoder of the s-VAE generates one or more conditional novel small molecules.

5. The processor-implemented method (200) of claim 1, wherein the one or more conditional novel small molecules is passed through one or more physico-chemical filters to satisfy one or more drug-like properties.

6. A system (100) comprising:

an input/output interface (104) to receive a gene expression profile in a cell-specific manner as an input and a dataset of molecules from a predefined drug-like small molecule database;
a memory (110) in communication with the one or more hardware processors (108), wherein the one or more hardware processors (108) are configured to execute programmed instructions stored in the memory (110) to:

pre-process the received dataset of molecules to obtain a training dataset of molecules with a predefined length, wherein the received dataset of molecules is pre-processed using filters to remove stereochemistry, salts, and molecules with undesirable atoms;
jointly train a simplified molecular input line entry system (SMILES) variational autoencoder (s-VAE) and a profile variational autoencoder (p-VAE) with the obtained training dataset of molecules, wherein the training dataset of molecules are represented in a SMILES format where grammar of the training dataset of molecules is learnt using the s-VAE; and
generate one or more conditional novel small molecules in SMILES format from the received gene expression profile using trained s-VAE and p-VAE, wherein during the joint training, a difference between the one or more conditional novel small molecules and the obtained training dataset of molecules is calculated as a joint loss function composed of binary cross entropy loss and Kullback-Leibler divergence (KLD) loss and wherein the joint loss function is backpropagated to a decoder that learns conditional mapping of a molecular space to a transcriptomic space in a cell-specific manner.

7. The system (100) of claim 6, wherein the gene expression profile reflects molecular signature of a disease.

8. The system (100) of claim 6, wherein the simplified molecular input line entry system (SMILES) is a string-based representation of the molecules.

9. The system (100) of claim 6, wherein an encoder of the p-VAE learns to project the received gene expression profiles into a latent space, and wherein the decoder of the s-VAE generates one or more conditional novel small molecules.

10. The system (100) of claim 6, wherein the one or more conditional novel small molecules is passed through one or more physico-chemical filters to satisfy one or more drug-like properties.

11. One or more non-transitory machine-readable information storage mediums comprising one or more instructions which when executed by one or more hardware processors cause:

receiving, via an input/output interface, a gene expression profile in a cell-specific manner as an input and a dataset of molecules from a predefined drug-like small molecule database;
pre-processing the received dataset of molecules to obtain a training dataset of molecules with a predefined length, wherein the received dataset of molecules is pre-processed using filters to remove stereochemistry, salts, and molecules with undesirable atoms;
jointly training a simplified molecular input line entry system (SMILES) variational autoencoder (s-VAE) and a profile variational autoencoder (p-VAE) with the obtained training dataset of molecules, wherein the training dataset of molecules are represented in a SMILES format where grammar of the training dataset of molecules is learnt using the s-VAE; and
generating one or more conditional novel small molecules in SMILES format from the received gene expression profile using trained s-VAE and p-VAE, wherein during the joint training, a difference between the one or more conditional novel small molecules and the obtained training dataset of molecules is calculated as a joint loss function composed of binary cross entropy loss and Kullback-Leibler divergence (KLD) loss and wherein the joint loss function is backpropagated to a decoder that learns conditional mapping of a molecular space to a transcriptomic space in a cell-specific manner.

12. The one or more non-transitory machine-readable information storage mediums of claim 11, wherein the gene expression profile reflects molecular signature of a disease.

13. The one or more non-transitory machine-readable information storage mediums of claim 11, wherein the one or more conditional novel small molecules is passed through one or more physico-chemical filters to satisfy one or more drug-like properties.

**Patentansprüche**

1. Prozessorimplementiertes Verfahren (200), umfassend:

   Empfangen (202), über eine Eingabe-/Ausgabe-Schnittstelle (104), eines Genexpressionsprofils auf eine zellspezifische Weise als eine Eingabe und eines Datensatzes von Molekülen aus einer vordefinierten arzneimittelartigen Kleinmolekül-Datenbank;
   Vorverarbeiten (204), über einen oder mehrere Hardwareprozessoren (108), des empfangenen Datensatzes von Molekülen, um einen Trainingsdatensatz von Molekülen mit einer vordefinierten Länge zu erhalten, wobei der empfangene Datensatz von Molekülen unter Verwendung von Filtern vorverarbeitet wird, um Stereochemie, Salze und Moleküle mit unerwünschten Atomen zu entfernen;
   gemeinsames Trainieren (206), über den einen oder die mehreren Hardwareprozessoren (108), eines Variationsautoencoders (s-VAE) eines vereinfachten molekularen Eingabelinieneingabesystems (SMILES) und eines Profilvariationsautoencoders (p-VAE) mit dem erhaltenen Trainingsdatensatz von Molekülen, wobei der Trainingsdatensatz von Molekülen in einem SMILES-Format dargestellt wird, wobei Grammatik des Trainingsdatensatzes von Molekülen unter Verwendung des s-VAE gelernt wird; und
   Erzeugen (208), über den einen oder die mehreren Hardwareprozessoren (108), eines oder mehrerer bedingter neuartiger kleiner Moleküle im SMILES-Format aus dem empfangenen Genexpressionsprofil unter Verwendung von trainiertem s-VAE und p-VAE, wobei während des gemeinsamen Trainings eine Differenz zwischen dem einen oder den mehreren bedingten neuartigen kleinen Molekülen und dem erhaltenen Trainingsdatensatz von Molekülen als eine gemeinsame Verlustfunktion berechnet wird, die aus binärem Kreuzentropie-Verlust und Kullback-Leibler-Divergenz (KLD)-Verlust besteht, und wobei die gemeinsame Verlustfunktion zu einem Decoder rückpropagiert wird, der bedingte Abbildung eines molekularen Raums auf einen transkriptomischen Raum auf eine zellspezifische Weise lernt.

2. Prozessorimplementiertes Verfahren (200) nach Anspruch 1, wobei das Genexpressionsprofil molekulare Signatur einer Krankheit widerspiegelt.

3. Prozessorimplementiertes Verfahren (200) nach Anspruch 1, wobei das vereinfachte molekulare Eingabelinieneingabesystem (SMILES) eine String-basierte Darstellung der Moleküle ist.

4. Prozessorimplementiertes Verfahren (200) nach Anspruch 1, wobei ein Codierer des p-VAE lernt, die empfangenen Genexpressionsprofile in einen latenten Raum zu projizieren, und wobei der Decoder des s-VAE ein oder mehrere bedingte neuartige kleine Moleküle erzeugt.

5. Prozessorimplementiertes Verfahren (200) nach Anspruch 1, wobei das eine oder die mehreren bedingten neuartigen kleinen Moleküle durch einen oder mehrere physikalisch-chemische Filter geleitet werden, um eine oder mehrere arzneimittelähnliche Eigenschaften zu erfüllen.

6. System (100), umfassend:

   eine Eingabe-/Ausgabe-Schnittstelle (104) zum Empfangen eines Genexpressionsprofils auf eine zellspezifische Weise als eine Eingabe und eines Datensatzes von Molekülen aus einer vordefinierten arzneimittelartigen Kleinmolekül-Datenbank;
   einen Speicher (110) in Kommunikation mit dem einen oder den mehreren Hardware-Prozessoren (108), wobei der eine oder die mehreren Hardware-Prozessoren (108) konfiguriert sind, um programmierte Anweisungen auszuführen, die in dem Speicher (110) gespeichert sind, um:

   den empfangenen Datensatz von Molekülen vorzuverarbeiten, um einen Trainingsdatensatz von Molekülen mit einer vordefinierten Länge zu erhalten, wobei der empfangene Datensatz von Molekülen unter Verwendung von Filtern vorverarbeitet wird, um Stereochemie, Salze und Moleküle mit unerwünschten Atomen zu entfernen;
   gemeinsam einen Variationsautoencoder (s-VAE) eines vereinfachten molekularen Eingabelinieneingabesystems (SMILES) und einen Profilvariationsautoencoder (p-VAE) mit dem erhaltenen Trainingsdatensatz von Molekülen zu trainieren,

wobei der Trainingsdatensatz von Molekülen in einem SMILES-Format dargestellt wird, wobei Grammatik des Trainingsdatensatzes von Molekülen unter Verwendung des s-VAE gelernt wird; und
ein oder mehrere bedingte neuartige kleine Moleküle im SMILES-Format aus dem empfangenen Genexpressionsprofil unter Verwendung von trainiertem s-VAE und p-VAE zu erzeugen, wobei während des gemeinsamen Trainings eine Differenz zwischen dem einen oder den mehreren bedingten neuartigen kleinen Molekülen und dem erhaltenen Trainingsdatensatz von Molekülen als eine gemeinsame Verlustfunktion berechnet wird, die aus binärem Kreuzentropie-Verlust und Kullback-Leibler-Divergenz (KLD)-Verlust besteht, und wobei die gemeinsame Verlustfunktion zu einem Decoder rückpropagiert wird, der bedingte Abbildung eines molekularen Raums auf einen transkriptomischen Raum auf eine zellspezifische Weise lernt.

7. System (100) nach Anspruch 6, wobei das Genexpressionsprofil molekulare Signatur einer Krankheit widerspiegelt.

8. System (100) nach Anspruch 6, wobei das vereinfachte molekulare Eingabelinieneingabesystem (SMILES) eine String-basierte Darstellung der Moleküle ist.

9. System (100) nach Anspruch 6, wobei ein Codierer des p-VAE lernt, die empfangenen Genexpressionsprofile in einen latenten Raum zu projizieren, und wobei der Decoder des s-VAE ein oder mehrere bedingte neuartige kleine Moleküle erzeugt.

10. System (100) nach Anspruch 6, wobei das eine oder die mehreren bedingten neuartigen kleinen Moleküle durch einen oder mehrere physikalisch-chemische Filter geleitet werden, um eine oder mehrere arzneimittelähnliche Eigenschaften zu erfüllen.

11. Ein oder mehrere nichtflüchtige maschinenlesbare Informationsspeichermedien, umfassend eine oder mehrere Anweisungen, die, wenn sie von einem oder mehreren Hardware-Prozessoren ausgeführt werden, bewirken:

Empfangen, über eine Eingabe-/Ausgabe-Schnittstelle, eines Genexpressionsprofils auf eine zellspezifische Weise als eine Eingabe und eines Datensatzes von Molekülen aus einer vordefinierten arzneimittelartigen Kleinmolekül-Datenbank;
Vorverarbeiten des empfangenen Datensatzes von Molekülen, um einen Trainingsdatensatz von Molekülen mit einer vordefinierten Länge zu erhalten, wobei der empfangene Datensatz von Molekülen unter Verwendung von Filtern vorverarbeitet wird, um Stereochemie, Salze und Moleküle mit unerwünschten Atomen zu entfernen;
gemeinsames Trainieren eines Variationsautoencoders (s-VAE) eines vereinfachten molekularen Eingabelinieneingabesystems (SMILES) und eines Profilvariationsautoencoders (p-VAE) mit dem erhaltenen Trainingsdatensatz von Molekülen, wobei der Trainingsdatensatz von Molekülen in einem SMILES-Format dargestellt wird, wobei Grammatik des Trainingsdatensatzes von Molekülen unter Verwendung des s-VAE gelernt wird; und
Erzeugen eines oder mehrerer bedingter neuartiger kleiner Moleküle im SMILES-Format aus dem empfangenen Genexpressionsprofil unter Verwendung von trainiertem s-VAE und p-VAE, wobei während des gemeinsamen Trainings eine Differenz zwischen dem einen oder den mehreren bedingten neuartigen kleinen Molekülen und dem erhaltenen Trainingsdatensatz von Molekülen als eine gemeinsame Verlustfunktion berechnet wird, die aus binärem Kreuzentropie-Verlust und Kullback-Leibler-Divergenz (KLD)-Verlust besteht, und wobei die gemeinsame Verlustfunktion zu einem Decoder rückpropagiert wird, der bedingte Abbildung eines molekularen Raums auf einen transkriptomischen Raum auf eine zellspezifische Weise lernt.

12. Ein oder mehrere nichtflüchtige maschinenlesbare Informationsspeichermedien nach Anspruch 11, wobei das Genexpressionsprofil molekulare Signatur einer Krankheit widerspiegelt.

13. Ein oder mehrere nichtflüchtige maschinenlesbare Informationsspeichermedien nach Anspruch 11, wobei das eine oder die mehreren bedingten neuartigen kleinen Moleküle durch einen oder mehrere physikalisch-chemische Filter geleitet werden, um eine oder mehrere arzneimittelähnliche Eigenschaften zu erfüllen.

## Revendications

1. Procédé mis en œuvre par processeur (200) comprenant :

la réception (202), via une interface d'entrée/sortie (104), d'un profil d'expression génique d'une manière spécifique à une cellule en tant qu'entrée et d'un ensemble de données de molécules à partir d'une base de données de petites molécules de type médicament prédéfinie ;

le prétraitement (204), via un ou plusieurs processeurs matériels (108), de l'ensemble de données de molécules reçu pour obtenir un ensemble de données d'apprentissage de molécules avec une longueur prédéfinie, dans lequel l'ensemble de données de molécules reçu est prétraité à l'aide de filtres pour éliminer la stéréochimie, les sels et les molécules avec des atomes indésirables ;

l'apprentissage conjoint (206), via les un ou plusieurs processeurs matériels (108), d'un autoencodeur variationnel (s-VAE) de système d'entrée de ligne d'entrée moléculaire simplifié (SMILES) et d'un autoencodeur variationnel de profil (p-VAE) avec l'ensemble de données d'apprentissage de molécules obtenu, dans lequel l'ensemble de données d'apprentissage de molécules est représenté dans un format SMILES où la grammaire de l'ensemble de données d'apprentissage de molécules est apprise à l'aide du s-VAE ; et

la génération (208), via les un ou plusieurs processeurs matériels (108), d'une ou plusieurs nouvelles petites molécules conditionnelles dans un format SMILES à partir du profil d'expression génique reçu à l'aide du s-VAE et du p-VAE entraînés, dans lequel pendant l'apprentissage conjoint, une différence entre les une ou plusieurs nouvelles petites molécules conditionnelles et l'ensemble de données d'apprentissage de molécules obtenu est calculée en tant que fonction de perte conjointe composée d'une perte d'entropie croisée binaire et d'une perte de divergence de Kullback-Leibler (KLD) et dans lequel la fonction de perte conjointe est rétropropagée vers un décodeur qui apprend un mappage conditionnel d'un espace moléculaire à un espace transcriptomique d'une manière spécifique à une cellule.

2. Procédé mis en œuvre par processeur (200) selon la revendication 1, dans lequel le profil d'expression génique reflète la signature moléculaire d'une maladie.

3. Procédé mis en œuvre par processeur (200) selon la revendication 1, dans lequel le système d'entrée de ligne d'entrée moléculaire simplifié (SMILES) est une représentation sous forme de chaîne des molécules.

4. Procédé mis en œuvre par processeur (200) selon la revendication 1, dans lequel un codeur du p-VAE apprend à projeter les profils d'expression génique reçus dans un espace latent, et dans lequel le décodeur du s-VAE génère une ou plusieurs nouvelles petites molécules conditionnelles.

5. Procédé mis en œuvre par processeur (200) selon la revendication 1, dans lequel les une ou plusieurs nouvelles petites molécules conditionnelles sont passées à travers un ou plusieurs filtres physico-chimiques pour satisfaire une ou plusieurs propriétés de type médicament.

6. Système (100) comprenant :

une interface d'entrée/sortie (104) pour recevoir un profil d'expression génique d'une manière spécifique à une cellule en tant qu'entrée et un ensemble de données de molécules à partir d'une base de données de petites molécules de type médicament prédéfinie ;

une mémoire (110) en communication avec les un ou plusieurs processeurs matériels (108), dans lequel les un ou plusieurs processeurs matériels (108) sont configurés pour exécuter des instructions programmées stockées dans la mémoire (110) pour :

prétraiter l'ensemble de données de molécules reçu pour obtenir un ensemble de données d'apprentissage de molécules avec une longueur prédéfinie, dans lequel l'ensemble de données de molécules reçu est prétraité à l'aide de filtres pour éliminer la stéréochimie, les sels et les molécules avec des atomes indésirables ;

entraîner conjointement un autoencodeur variationnel (s-VAE) de système d'entrée de ligne d'entrée moléculaire simplifié (SMILES) et un autoencodeur variationnel de profil (p-VAE) avec l'ensemble de données d'apprentissage de molécules obtenu, dans lequel l'ensemble de données d'apprentissage de molécules est représenté dans un format SMILES où la grammaire de l'ensemble de données d'apprentissage de molécules est apprise à l'aide du s-VAE ; et

générer une ou plusieurs nouvelles petites molécules conditionnelles dans un format SMILES à partir du profil d'expression génique reçu à l'aide du s-VAE et du p-VAE entraînés, dans lequel pendant l'apprentissage conjoint, une différence entre les une ou plusieurs nouvelles petites molécules conditionnelles et l'ensemble de données d'apprentissage de molécules obtenu est calculée en tant que fonction de perte conjointe composée d'une perte d'entropie croisée binaire et d'une perte de divergence de Kullback-Leibler (KLD) et dans lequel la fonction de perte conjointe est rétropropagée vers un décodeur qui apprend un mappage conditionnel d'un espace moléculaire à un espace transcriptomique d'une manière spécifique à une cellule.

7. Système (100) selon la revendication 6, dans lequel le profil d'expression génique reflète la signature moléculaire d'une maladie.

8. Système (100) selon la revendication 6, dans lequel le système d'entrée de ligne d'entrée moléculaire simplifié (SMILES) est une représentation sous forme de chaîne des molécules.

9. Système (100) selon la revendication 6, dans lequel un codeur du p-VAE apprend à projeter les profils d'expression génique reçus dans un espace latent, et dans lequel le décodeur du s-VAE génère une ou plusieurs nouvelles petites molécules conditionnelles.

10. Système (100) selon la revendication 6, dans lequel les une ou plusieurs nouvelles petites molécules conditionnelles sont passées à travers un ou plusieurs filtres physico-chimiques pour satisfaire une ou plusieurs propriétés de type médicament.

11. Un ou plusieurs supports de stockage d'informations lisibles par machine non transitoires comprenant une ou plusieurs instructions qui, lorsqu'elles sont exécutées par un ou plusieurs processeurs matériels, amènent :

la réception, via une interface d'entrée/sortie, d'un profil d'expression génique d'une manière spécifique à une cellule en tant qu'entrée et d'un ensemble de données de molécules à partir d'une base de données de petites molécules de type médicament prédéfinie ;

le prétraitement de l'ensemble de données de molécules reçu pour obtenir un ensemble de données d'apprentissage de molécules avec une longueur prédéfinie, dans lequel l'ensemble de données de molécules reçu est prétraité à l'aide de filtres pour éliminer la stéréochimie, les sels et les molécules avec des atomes indésirables ;

l'entraînement conjoint d'un autoencodeur variationnel (s-VAE) de système d'entrée de ligne d'entrée moléculaire simplifié (SMILES) et d'un autoencodeur variationnel de profil (p-VAE) avec l'ensemble de données d'apprentissage de molécules obtenu, dans lequel l'ensemble de données d'apprentissage de molécules est représenté dans un format SMILES où la grammaire de l'ensemble de données d'apprentissage de molécules est apprise à l'aide du s-VAE ; et

la génération d'une ou de plusieurs nouvelles petites molécules conditionnelles dans un format SMILES à partir du profil d'expression génique reçu à l'aide du s-VAE et du p-VAE entraînés, dans lequel pendant l'apprentissage conjoint, une différence entre les une ou plusieurs nouvelles petites molécules conditionnelles et l'ensemble de données d'apprentissage de molécules obtenu est calculée en tant que fonction de perte conjointe composée d'une perte d'entropie croisée binaire et d'une perte de divergence de Kullback-Leibler (KLD) et dans lequel la fonction de perte conjointe est rétropropagée vers un décodeur qui apprend un mappage conditionnel d'un espace moléculaire à un espace transcriptomique d'une manière spécifique à une cellule.

12. Un ou plusieurs supports de stockage d'informations lisibles par machine non transitoires selon la revendication 11, dans lequel le profil d'expression génique reflète la signature moléculaire d'une maladie.

13. Un ou plusieurs supports de stockage d'informations lisibles par machine non transitoires selon la revendication 11, dans lequel les une ou plusieurs nouvelles petites molécules conditionnelles sont passées à travers un ou plusieurs filtres physico-chimiques pour satisfaire une ou plusieurs propriétés de type médicament.

100

104-1

104-2

104-N

106

102

108

110

112

**FIG. 1**

200

Receiving a gene expression profile in a cell-specific manner as an input and a dataset of molecules from a predefined drug-like small molecule database — 202

Pre-processing the received dataset of molecules to obtain a training dataset of molecules — 204

Training a simplified molecular input line entry system (SMILES) variational autoencoder (s-VAE) and a profile variational autoencoder (p-VAE) jointly with the obtained training dataset of molecules — 206

Generating one or more conditional novel small molecules in SMILES format from the received gene expression profile using jointly trained s-VAE and p-VAE — 208

FIG. 2

300

The RNA sequencing data is obtained for samples in the disease state and control. — 302

Differential gene expression analysis is carried out using characteristic direction method. — 304

The differential gene expression profile of the disease state is reversed to obtain the desired gene expression profile — 306

Small molecules are generated using pre-trained deep learning model — 308

Drug candidates are screened by applying biological filters. — 310

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JANNIS et al.** *PaccMann^RL: De novo generation of hit-like anticancer molecules from transcriptomic data via reinforcement learning*